(19) Europäisches Patentamt
European Patent Office
Office européen des brevets

(11) **EP 4 047 355 A1**

(12) **EUROPEAN PATENT APPLICATION**

(43) Date of publication:
**24.08.2022 Bulletin 2022/34**

(21) Application number: **21382142.4**

(22) Date of filing: **23.02.2021**

(51) International Patent Classification (IPC):
**G01N 22/00** *(2006.01)*    **G01N 27/22** *(2006.01)*

(52) Cooperative Patent Classification (CPC):
**G01N 22/00; G01N 27/221; G01N 33/483**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitat Politècnica De Catalunya 08034 Barcelona (ES)**

(72) Inventors:
• **JOFRE CRUANYES, Marc 08015 Barcelona (ES)**

• **JOFRE CRUANYES, Lluís 08360 Barcelona (ES)**
• **JOFRE ROCA, Luis 08360 Barcelona (ES)**
• **PALACIOS ARIAS, César Augusto 08520 Barcelona (ES)**
• **ROMEU ROBERT, Jordi 08173 Barcelona (ES)**
• **O CALLAGHAN CASTELLÀ, Juan Manuel 08029 Barcelona (ES)**

(74) Representative: **Segui Quetglas, Margalida et al Torner, Juncosa i Associats, S.L. C/Pau Claris, 108, 1r 1a 08009 Barcelona (ES)**

(54) **A METHOD AND A SYSTEM FOR DETECTING MICROWAVE SIGNALS WHICH CARRY INFORMATION OF THE FUNCTIONAL DYNAMICS OF BIOLOGICAL PARTICLES**

(57)    A method and a system for detecting microwave signals which carry information of the functional dynamics of biological particles are provided. The method comprises illuminating a biological particle with microwave electromagnetic waves, the microwave electromagnetic waves being comprised in a non-thermal intensity regime, having frequencies with wavelengths much larger than characteristic dimensions of the biological particle and being adapted to a functional dynamics signature of the biological particle; adapting the illuminating microwave electromagnetic waves to produce additional frequencies by a process of frequency mixing in a membrane of the biological particle with a nonlinear relationship; capturing a response affected by the nonlinear relationship at different functional states of the biological particle; and detecting resulting frequency components of the captured response by means of a filtering element, so as to thereby infer the functional dynamics of the biological particle.

**Fig. 2**

## Description

### Technical Field

The present invention relates to measurements of the functional dynamics of biological particles. In particular, the invention relates to the measurement of the membrane potential of biological particles with microwave frequency mixing adapted to the characteristic signal signature of the membrane making use of the nonlinearity of the voltage-current response of the membrane of biological particles.

### Background of the Invention

In neurons (eukaryotic cells) the action potential is used in order to transmit information to other neurons, whilst in bacteria biofilms' equivalent action potentials are generated to transmit information to external, or other bacteria, in the surrounding. The two systems are behaviorally similar, but quorum sensing in bacteria is more easily studied in depth than cell-cell signaling in eukaryotes [1]. Recent microbiology advances have determined that bacteria are an easily characterizable model organism with an extraordinarily complicated set of abilities, comparable cells or to the whole brain sensing [2], [3]. Among them is quorum sensing, a cell-cell signaling system that may have a common evolutionary origin with eukaryotic cell-cell signaling, and related to action potentials. Because of this comparative ease of study, bacterial dynamics are also more suited to direct interpretation than eukaryotic dynamics, i.e., those of the neuron [4].

The term action potential refers to the electrical signaling that occurs within neurons or bacteria biofilms, arising from changes in membrane potential from ion concentrations in the vicinity of membranes. An action potential is a rapid rise and subsequent fall in voltage, or membrane potential, across a cellular membrane with a characteristic pattern. Sufficient current is required to initiate a voltage response in a cell membrane; if the current is insufficient to depolarize the membrane to the threshold level, an action potential will not be triggered [5], [6]. Membrane potential refers to the difference in charge between the inside and outside of a cell (such as neurons or bacteria), which is created due to the unequal distribution of ions on both sides of the cell.

The plasma membrane of a cell typically has a transmembrane potential of approximately -100mV (negative inside) as a consequence of $k+$, $Na+$ and $Cl-$ concentration gradients that are maintained by active transport processes. Typically it is detected by means of optical techniques using biological markers [7], [8]. For instance based on optogenetics by incorporating into an electrically excitable cell an optical reporter [10], [11]. A signal is obtained from the optical reporter in response to a stimulation of the cell. In particular, potentiometric optical probes enable membrane potential measurements in organelles and in bacterial cells that are too small for microelectrodes. Potentiometric probes are an indirect method of detecting the translocation of these ions, whereas the fluorescent ion indicators can be used to directly measure changes in specific ion concentrations. Moreover, in conjunction with imaging techniques, these probes can be employed to map variations in membrane potential across excitable cells, with spatial resolution and sampling frequency that cannot be obtained using microelectrodes. Furthermore, patch-clamp technique allows direct measurements of electrical potential but it is highly invasive . Patch-clamp technique has been applied only with isolated bacterial membranes or giant spheroplasts and protoplasts. Therefore, while patch clamp is a powerful technique for studying prokaryotic ion channels, it is not suitable for functional studies of membrane potential dynamics under physiological conditions. Instead, very little intrusive methods for measuring bio-particle membrane potentials has been demonstrated using Second Harmonic Generation (SHG) with optical techniques [9], that make use of the susceptibility tensor of the membrane. SHG not only can monitor the movement of small molecules across membrane leaflets but also is sensitive to higher-level ordering of the molecules within the membrane.

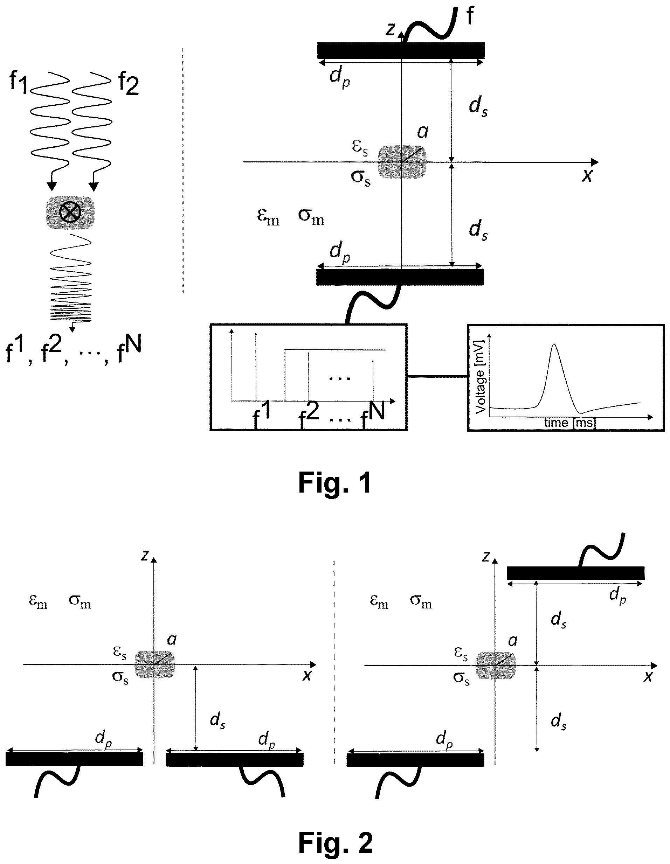

In many situations, electromagnetic waves interact with inhomogeneous particles and the corresponding electromagnetic-matter interaction process is referred to as scattering. A general scheme of the configuration of electromagnetic illumination and detection of scattering by a small bio-particle (e.g., bacteria) in a medium (e.g., water) is depicted in Fig. 1. Here, particle refers to a region in space characterized by a dielectric complex permittivity ($\varepsilon_s$, $\sigma_s$) which is different from that of the surrounding medium ($\varepsilon_m, \sigma_m$).

At microwave frequencies and for the typical dimensions of the experimental setups, the conditions are of near-field. In particular, the bio-particle is isolated due to using dilute enough solutions, only is considered single scattering (not considering multiple scattering) as described in [12]-[14]. But, in a general situation also considering non-dilute bio-particles (e.g., inside the brain), the same physical principles would apply and multiple-scattering would be considered.

### Scattering by a small spherical particle

When an electromagnetic wave impinges on an object, the incident wave is modeled as a plane electromagnetic wave (sufficiently accurate since the emitter is much larger than the particle). Assuming the electromagnetic wave to be polarized in the z direction and propagating in the x direction. Also assuming the particle to be a small spherical particle with permittivity $\varepsilon_s$ and radius a. Essentially, the particle sees a constant field as the plane wave impinges on it (and almost electrostatic field on the incident field). The incident field polarizes the particle making it look like an electric dipole. Since the incident field is a time harmonic, the small electric dipole will oscillate and radiate like a Hertzian dipole in the far field, but it can also be analysed in the near-field. A Hertzian dipole can be approximated by a small current source so that $\vec{J}(\vec{r}) = \hat{z}Il\delta(\vec{r})$.

*Near-Field regime (a similar analysis could be performed for far-field regimes)*

The electric field is given by $\vec{E} = -j\omega\vec{A} - \nabla\Phi$, where the scalar potential term dominates over the vector potential in the near field of the scatterer. The scalar potential $\Phi(\vec{r})$ is obtained from the Lorenz gauge as $\nabla \cdot \vec{A} = -j\omega\mu\varepsilon\Phi$, resulting in

$$\Phi(\vec{r}) = \frac{-1}{j\omega\mu\varepsilon}\nabla \cdot \vec{A} = \frac{-Il}{j\omega\varepsilon 4\pi}\frac{\partial}{\partial z}\frac{1}{r}e^{-j\beta r}.$$

When close to the dipole, by assuming that $\beta r \ll 1$, a quasi-static approximation can be utilized for the potential (equivalent to ignoring retardation effect)

$$\frac{\partial}{\partial z}\frac{1}{r}e^{-j\beta r} \approx \frac{-\cos\theta}{r^2},$$

and reducing the potential to

$$\Phi(\vec{r}) \approx \frac{ql}{4\pi\epsilon r^2}\cos\theta.$$

This dipole induced in the small particle is formed in response to the incident field. The incident field can be approximated by a constant local static electric field $\vec{E}_{inc} = \hat{z}\,E_i$. The corresponding electrostatic potential for the incident field is then $\Phi_{inc} = -\hat{z}\,E_i$, so that $\vec{E}_{inc} \approx -\nabla\Phi_{inc} = \hat{z}E_i$. The scattered dipole potential from the spherical particle in the vicinity of it is given by

$$\Phi_{sca} = E_s\frac{a^3}{r^2}\cos\theta.$$

The electrostatic boundary problem is $E_s = \frac{\epsilon_s - \epsilon}{\epsilon_s + 2\epsilon}E_i$. As a result, the electric scattered field is given by $\vec{E}_{sca} = \left[\frac{k^2 a^3}{r}\frac{\epsilon_s - \epsilon}{\epsilon_s + 2\epsilon} - \frac{a^3}{r^3}\frac{\epsilon_s - \epsilon}{\epsilon_s + 2\epsilon}\right]E_i\,\hat{z} \approx -\frac{a^3}{r^3}\frac{\epsilon_s - \epsilon}{\epsilon_s + 2\epsilon}E_i\,\hat{z}.$

A similar derivation can be obtained for the magnetic scattered near-field given by $\vec{H}_{sca} \approx \frac{k\,a^3}{r^2}\frac{\epsilon_s - \epsilon}{\epsilon_s + 2\epsilon}H_i\,\hat{z},$ where $\vec{H}_{inc} = \hat{z}\,H_i$ is the incident magnetic field.

The complex permittivity of biological particles with a membrane at rest, or when generating a potential, do not differ much from each state. The membrane's capacitance (related to the real part of the complex permittivity) of bacteria depends on its surface area, while the conductivity of the membrane depends on its cross-sectional area [15]. Hence, it is not expected to easily observe a large variation between a biological particle at rest and at generating an action potential by making use of typical linear electromagnetic techniques, because these physical dimensions of the biological particles are not expected to have a major change.

Description of the Invention

An object of the present invention is thus to provide a microwave sensing configuration for membrane potential measurement which overcomes the limitations of the prior art. For this purpose, the invention comprises the steps of exciting microwave oscillating potentials in order to produce mixing of the microwave illuminating electromagnetic field at given frequencies within the membrane of biological particles, which will generate a scattered field at the fundamental frequency and also at harmonics related to the non-linear relation between the voltage and current in the membrane of biological particles.

This object is fulfilled by a method with the characteristics of claim 1 and with a system with the characteristics of claim 7.

Embodiments of the present invention provide according to a first aspect a method for detecting microwave signals which carry information of the functional dynamics of biological particles. The method comprises illuminating one or more biological particles with microwave electromagnetic waves, the microwave electromagnetic waves being comprised in a non-thermal intensity regime, having frequencies with wavelengths much larger than characteristic dimensions of the biological particle(s) and being adapted to a functional dynamics signature of the biological particle(s); adapting/transforming the illuminating microwave electromagnetic waves to produce additional frequencies (i.e. harmonics) by a process of frequency mixing in the membrane of the biological particle(s) with a nonlinear relationship; capturing a response affected by the nonlinear relationship at different functional states of the biological particle(s); and detecting resulting frequency components of the captured response by means of a filtering element, so as to thereby infer the functional dynamics of the biological particle(s).

Particularly, the nonlinear relationship is a nonlinear current-voltage relationship.

In an embodiment, the illuminating microwave electromagnetic waves are operated in power far below a threshold for thermal damaging the biological particle(s). Alternatively or complementarily, the illuminating microwave electromagnetic waves are operated such that the duration of the illumination is adapted to the temporal functional dynamics of the biological particle(s). Yet, alternatively or complementarily, the frequencies of the illuminating microwave electromagnetic waves can be adjusted to resonances of the dynamics of the biological particle(s).

In an embodiment, the method further comprises a step of isolating the frequencies of the illuminating microwave electromagnetic waves, either spatially or in frequency.

Embodiments of the present invention also provide according to a second aspect a system for detecting microwave signals which carry information of the functional dynamics of biological particles. The system comprises a biological particle, a microwave generator, an illumination adapter/controller, a microwave detector and a filter element.

The microwave generator is adapted/configured to produce microwave electromagnetic waves to illuminate the biological particle, the microwave electromagnetic waves being comprised in a non-thermal intensity regime, having frequencies with wavelengths much larger than characteristic dimensions of the biological particle and being adapted to a functional dynamics signature of the biological particle. The illumination adapter is adapted/configured to adapt the illuminating microwave electromagnetic waves to produce additional frequencies through a process of frequency mixing in a membrane of the biological particle with a nonlinear relationship. The microwave detector is adapted/configured to capture a response affected by the nonlinear relationship at different functional states of the biological particle. The filter element is adapted/configured to detect resulting frequency components of the captured response.

In an embodiment, the system also includes a spatial or frequency isolating element to isolate the frequencies of the illuminating microwave electromagnetic waves.

In an embodiment, the microwave generator is adapted to be pulsed and operated such that the duration of the illumination is adjusted to the temporal functional dynamics of the biological particle.

The illumination adapter and the microwave detector can be positioned either collinear or adjacent to each other. In other embodiments, the illumination adapter can be arranged at one of the sides of the biological particle whereas the microwave detector is arranged at another side of the biological particle. In any case, both elements/units do not need to be directly confronted.

In an embodiment, the filter element is a high-pass frequency filter. In another embodiment the filter element comprises a matched filter receiver configured to exploit matched filter signal processing schemes, for example Passive Intermodulation Products, among others.

In an embodiment, the microwave detector is based on sensitive quantum electromagnetic detection schemes.

Brief Description of the Drawings

The previous and other advantages and features will be more fully understood from the following detailed description of embodiments, with reference to the attached figures, which must be considered in an illustrative and non-limiting manner, in which:

Fig. 1 is a general scheme of the configuration of electromagnetic illumination and detection of scattering by a small bio-particle (e.g., bacteria) in a medium (e.g., water). The electromagnetic response of bacteria depends on their shape and size, their internal structure, and the electric conductivity and permittivity of the different bacterial cell components, which may depend on the bacterial physiological state but depend very little on the functional state of the bacteria. Scheme of the configuration and relevant values depicted, where $\varepsilon_s$ stands for permittivity of the cell, $\sigma_s$ conductivity, $a$ radius, $\varepsilon_m$ permittivity of the medium, $\sigma_m$ conductivity, $d_p$ is the length of the illumination and detection plates and $d_s$ distance to the cell (considered equally spaced)

Fig. 2 shows different setups for putting into practice the process according to the invention, in which the detection element is positioned at different orientations with respect to the illumination element.

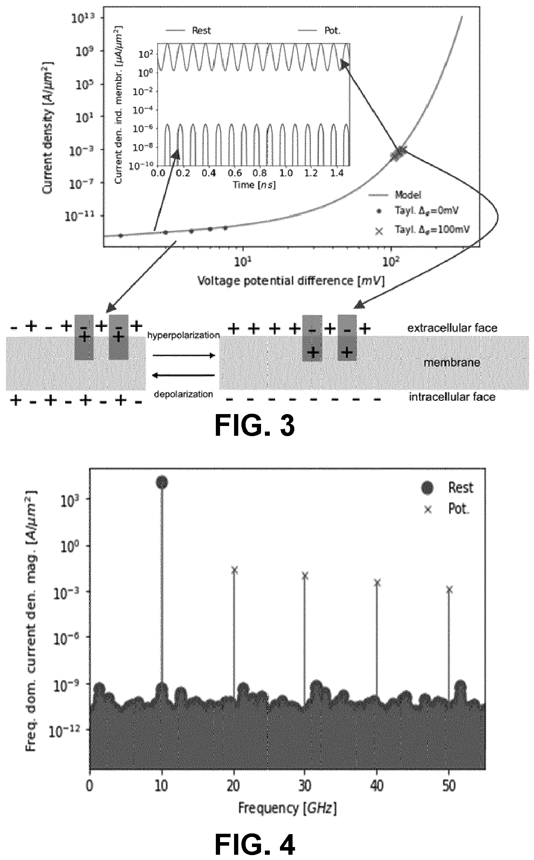

Fig. 3 illustrates the nonlinear voltage-current response of the membrane potential; (top) current density non-linear dependence with the differential voltage potential; (inset) induced current density due to an impinging microwave field at two different membrane potential states, while at rest and while at generating an action potential. The magnitudes of the induced current density differ in eight orders of magnitude; (bottom) shows a membrane scheme where fast-response operates by means of a change in their electronic structure, in response to a change in the surrounding electric field.

Fig. 4 illustrates the frequency domain analysis where the fundamental frequency (pump) scattered due to the bio-particle and its functional state is masked by the illumination itself, making it very difficult to sense the difference between the bacteria at rest and in an action potential state. Instead, the difference of the electromagnetic response of the bacteria in the two different functional states in the second frequency harmonic is around 8 orders of magnitude.

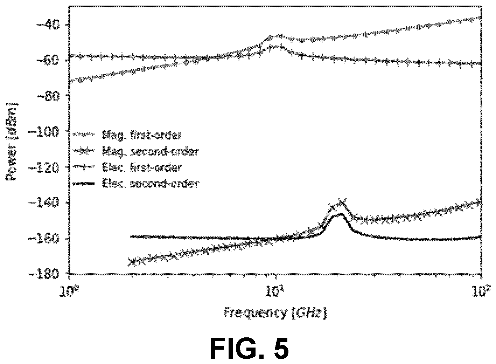

Fig. 5 shows that the signal levels regarding magnetic fields are slightly higher compared to electric fields for higher frequencies. Considering the magnetic or electric field, the second-order signal level is close to the noise level. Different techniques can be applied in order to improve the sensitivity of detectors, such as novel quantum electro-magnetic sensing schemes.

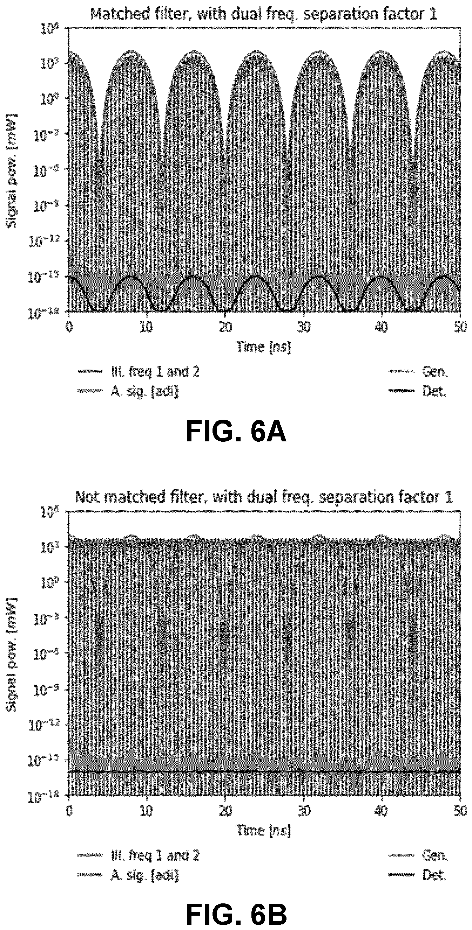

Figs. 6A and 6B show an example in which illumination with microwave signals is adapted to the signature of the functional dynamics of the biological particle, in order to detect these from the potential other signals present in the system and not carrying information of the functional dynamics of the biological particles.

## Detailed Description of Preferred Embodiments

Present invention is based on the transformation of the illuminating energy in the microwave illuminating frequencies in order to produce subsequent harmonics within the nonlinear current-voltage dependence in the membranes of biological particles.

More in particular, present invention provides a method and system for measuring membrane potential which carries information on the functional dynamics of biological particles by means of a microwave nonlinear scattering. The invention operates microwave signals by means of the signal signature of the dynamics of the biological particles, combined with the non-linear response of the membrane of microorganisms, transforms the frequencies of the incident illuminating microwave electromagnetic field in a sequence of harmonics, and detects the resulting signal. The signal detected is a proxy for the biological particles functional dynamics.

According to a first embodiment, as shown in Fig. 1, this is achieved by illuminating the biological particle with microwave electromagnetic wave at frequencies $f_1, f_2$...(e.g., *1GHz - 100GHz)*, where the different illumination frequencies can be indicated as *f*, where the specific intensities of the illuminating microwave signals in the non-thermal regime (e.g., *0dBm* - 30*dBm*) and frequencies (e.g., with impinging frequencies much larger than the equivalent characteristic dimension of the biological particle of $1\mu m$ - *1mm,* being $\Delta f$ in the order of 100*Hz* - 10*GHz)* are adjusted properly to produce the mixing of the frequencies, related by the principle of energy conservation $\sum f = \sum_{n=1}^{N} f^n$, within the membrane of the biological cell producing, with a relative high efficiency of transformation, several harmonics $f^1, f^2 \cdots, f^N$. Furthermore, higher order nonlinear terms become significant as voltage across the membrane of the biological particle is increased, such as in the process of generating an action potential.

Cellular as well as intracellular membranes exhibit a distinct nonlinear electrical behavior, due to the potential barrier resulting from the difference between inner and outer electrolytes and the action of ion-pumps [16]. In the absence of an action potential, the transmembrane potential difference $\Delta_\phi$ is equal to the cell resting potential $V_0$ ($\approx$ -100*mV*, in a typical cell). When an action potential occurs, a transmembrane excess potential appears $\Delta_\phi = V_0 + \delta_\phi$. As a result, a transmembrane current density $\vec{J_m}$ flows. The current-voltage response is known to be fairly well approximated by a nonlinear diode-like relationship of the form $J_m = J_0(e^{\delta_\phi / V_T} - 1)$ with typ-

ical values $J_0 \approx 10^{-5} A/cm^2$, $V_T \approx 5mV$ [17]-[21]. Accordingly, all electrical variables of interest (e.g., field, currents, etc.) may be derived from the occuring single scalar potential $\Phi(\vec{r})$.

Referring back to Fig. 1, the cell's membrane is illuminated with microwave fields while the bias working point in the non-linear voltage-current model will be determined by the biological particle self-potential state. Depending on the state of the membrane potential, the adjusted impinging microwave signals will scatter having multiple generated frequency harmonics (due to the nonlinear voltage-current model of the membrane), compared to the incident illuminating signals. In particular, using Taylor series expansion for the first two terms of the transmembrane current density $J_m$, the expected efficiency of the second-order process at $\delta_\phi$ = 100*mV*, compared to $\delta_\phi$ = 0*mV*, is $2 \cdot 10^8$ times larger, while the second-order process at $\delta_\phi$ = 100mV compared to the first-order is $3 \cdot 10^{-5}$, which is still considerably efficient.

When impinging with microwave frequencies fields (e.g., *10GHz),* induced currents density is generated on the working point of the membrane potential state, where its magnitude and frequency harmonics are incremented when the cell is at an action potential compared to a rest state. The illumination frequencies (pump) scattering due to the membrane (around 10*nm*) is obscured compared to the scattering generated by the cell's whole body (which is at least two orders of magnitude larger), making it very difficult to sense the difference between the bacteria at rest and in an action potential state. Instead, for instance for the second frequency harmonic there is around 11 orders of magnitude difference at the two cell's state, because it is essentially sensing directly the effect of the membrane potential and not the whole bacterial body. The expected relative second-order response at different frequencies, with respect to the fundamental pump frequency, at rest and at action potential state, are shown in Fig. 3. Furthermore, as known, the size of the bacteria scales the response with the cube of the radius, hence it is more challenging to detect $1\mu m$ than $10\mu m$ cells.

In Fig. 4 is shown the second harmonic generation signal level with respect the pump frequencies in the *GHz* range and the pump power in the *mW* range. It is shown, as it is common for frequency conversion, the non-linear relation between pump power and generated harmonics' power, while the dependence on pump frequency is smoother.

For typical room temperature and a bandwidth of *10Hz,* the noise level is around *-164dBm,* while at *4K* cryogenic temperature levels the equivalent noise is around *-182dBm.* Moreover, in the reactive near-field region of electromagnetic fields, the response levels of the electric and magnetic fields are not the same. In particular, the magnetic field, more closely related to currents, computes to have a higher level of electromagnetic response for higher frequencies, as shown in Fig. 5. Nevertheless, the expected intensity levels and the equivalent noise levels are of the same order. A cryogenic low noise amplifier, or other similar techniques, may be appropriate to experiment with to detect such low signal levels to achieve a reasonable SNR and enter into the sensitivity limits of detectors.

In Figs. 6A and 6B the illuminating microwave signals are adapted to the signature of the functional dynamics of the biological particle, in order to detect these from the potential other signals present in the system and not carrying information of the functional dynamics of the biological particles. Fig. 6A shows in the time domain the expected signal signature of the functional dynamics of the biological particles, the generated microwave signals adapted to the specific signature, the tailored generated signals from the functional dynamics of the biological particle and the detected signal carrying information of the functional dynamics of the biological particles. Fig. 6B shows in the time domain the expected signal signature of the functional dynamics of the biological particles, generated microwave signals not adapted to the specific signature, the generated signals from the functional dynamics of the biological particle and the not detected signal carrying information of the functional dynamics of the biological particles. Note that the signal signature is not scale, but not detrimental to the procedure, in order to easily show the steps described in this invention.

The scope of the present invention is defined in the following set of claims.

## References

[1] J. P. Stratford et al., "Electrically induced bacterial membrane-potential dynamics correspond to cellular proliferation capacity," PNAS, vol. 116, no. 19, Art. no. 19, May 2019, doi: 10.1073/pnas.1901788116.

[2] "The origins of the brain's endogenous electromagnetic field and its relationship to provision of consciousness | Journal of Integrative Neuroscience," Jul. 11, 2020. https://www.worldscientific.com/doi/abs/10.1142/S0219635214400056 (accessed Jul. 11, 2020).

[3] "The Big and the Small: Challenges of Imaging the Brain's Circuits | Science," Jul. 11, 2020. https://science.sciencemag.org/content/334/6056/618/tab-article-info (accessed Jul. 11, 2020).

[4] A. Ram and A. W. Lo, "Is Smaller Better? A Proposal to Use Bacteria For Neuroscientific Modeling," Front. Comput. Neurosci., vol. 12, 2018, doi: 10.3389/fncom.2018.00007.

[5] V. Pikov, X. Arakaki, M. Harrington, S. E. Fraser, and P. H. Siegel, "Modulation of neuronal activity and plasma membrane properties with low-power millimeter waves in organotypic cortical slices," J. Neural Eng., vol. 7, no. 4, Art. no. 4, Jul. 2010, doi: 10.1088/1741-2560/7/4/045003.

[6] M. N. Shneider and M. Pekker, "Initiation and blocking of the action potential in an axon in weak ultrasonic or microwave fields," Phys. Rev. E, vol. 89, no. 5, Art. no. 5, May 2014, doi: 10.1103/PhysRevE.89.052713.

[7] I. Johnson, et al., "The Molecular Probes Handbook, 11th Edition," 2010. //www.thermofisher.com/es/es/home/references/molecular-probes-the-handbook/mp-handbook-download.html.

[8] A. E. Cohen, et al., "Optogenetic probes for measuring membrane potential," US10352945B2, Jul. 16, 2019.

[9] L. N. Miller, et al., W. T. Brewer, J. D. Williams, E. M. Fozo, and T. R. Calhoun, "Second Harmonic Generation Spectroscopy of Membrane Probe Dynamics in Gram-Positive Bacteria," Biophysical Journal, vol. 117, no. 8, pp. 1419-1428, Oct. 2019, doi: 10.1016/j.bpj.2019.09.014.

[10] A. E. Cohen, et al., "Systems, methods, and workflows for optogenetics analysis," US10161937B2, Dec. 25, 2018.

[11] K. A. Deisseroth, et al., "Devices, systems and methods for optogenetic modulation of action potentials in target cells," US20190125871 A1, May 02, 2019.

[12] W. C. Chew, "Waves and fields in inhomogeneous media," CERN Document Server, 1995. https://cds.cern.ch/record/1480882 (accessed Dec. 12, 2020).

[13] C. A. Balanis, Advanced Engineering Electromagnetics. John Wiley & Sons, 2012.

[14] A. Ishimaru, Electromagnetic Wave Propagation, Radiation, and Scattering: From Fundamentals to Applications. John Wiley & Sons, 2017.

[15] J. M. Benarroch and M. Asally, "The Microbiologist's Guide to Membrane Potential Dynamics," Trends in Microbiology, vol. 28, no. 4, Art. no. 4, Apr. 2020, doi: 10.1016/j.tim.2019.12.008.

[16] "Does the resting potential of Chara braunii have an electrogenic component?" https://cdnsciencepub.com/doi/abs/10.1139/b73-089 (accessed Dec. 12, 2020).

[17] G. Franceschetti and I. Pinto, "Cell Membrane Nonlinear Response to an Applied Electromagnetic Field," IEEE Trans. Microwave Theory Techn., vol. 32, no. 7, pp. 653-658, Jul. 1984, doi: 10.1109/TMTT. 1984.1132749.

[18] A. L. Hodgkin and A. F. Huxley, "A quantitative description of membrane current and its application to conduction and excitation in nerve," J Physiol, vol. 117, no. 4, pp. 500-544, Aug. 1952.

[19] R. FitzHugh, "Impulses and Physiological States in Theoretical Models of Nerve Membrane," Biophys J, vol. 1, no. 6, pp. 445-466, Jul. 1961.

[20] E. M. Izhikevich, "Simple model of spiking neurons," IEEE Transactions on Neural Networks, vol.

14, no. 6, pp. 1569-1572, Nov. 2003, doi: 10.1109/TNN.2003.820440.

[21]P. Phillipson, "A Comparative Study of the Hodgkin-huxley and Fitzhugh-nagumo Models of Neuron Pulse Propagation.," I. J. Bifurcation and Chaos, vol. 15, pp. 3851-3866, Dec. 2005, doi: 10.1142/S0218127405014349.

**Claims**

1. A method for detecting microwave signals which carry information of the functional dynamics of biological particles, the method comprising the steps of:

   a) illuminating at least one biological particle with microwave electromagnetic waves, the microwave electromagnetic waves being comprised in a non-thermal intensity regime, having frequencies with wavelengths much larger than characteristic dimensions of the biological particle and being adapted to a functional dynamics signature of the biological particle;
   b) adapting the illuminating microwave electromagnetic waves to produce additional frequencies by a process of frequency mixing in a membrane of the biological particle with a nonlinear relationship;
   c) capturing a response affected by the nonlinear relationship at different functional states of the biological particle; and
   d) detecting resulting frequency components of the captured response by means of a filtering element,

   so as to thereby infer the functional dynamics of the biological particle.

2. The method according to claim 1, wherein the nonlinear relationship comprises a nonlinear current-voltage relationship.

3. The method according to claim 1 or 2, wherein the illuminating microwave electromagnetic waves are operated in power far below a threshold for thermal damaging the biological particle.

4. The method according to claim 1 or 2, wherein the illuminating microwave electromagnetic waves are operated such that a duration of the illumination is adapted to the temporal functional dynamics of the biological particle.

5. The process according to claim 1 or 2, wherein the frequencies of the illuminating microwave electromagnetic waves are adjusted to resonances of the dynamics of the biological particle.

6. The method according to any one of the previous claims, further comprising a step of isolating the fundamental frequencies of the illuminating microwave electromagnetic waves, either spatially or in frequency.

7. A system for detecting microwave signals which carry information of the functional dynamics of biological particles, comprising:

   at least one biological particle;
   a microwave generator configured to produce microwave electromagnetic waves to illuminate at least one biological particle, the microwave electromagnetic waves being comprised in a non-thermal intensity regime, having frequencies with wavelengths much larger than characteristic dimensions of the biological particle and being adapted to a functional dynamics signature of the biological particle;
   an illumination adapter configured to adapt the illuminating microwave electromagnetic waves to produce additional frequencies through a process of frequency mixing in a membrane of the biological particle with a nonlinear relationship;
   a microwave detector configured to capture a response affected by the nonlinear relationship at different functional states of the biological particle; and
   a filter element configured to detect resulting frequency components of the captured response.

8. The system according to claim 7, further comprising a spatial or frequency isolating element configured to isolate the fundamental frequencies of the illuminating microwave electromagnetic waves.

9. The system according to claim 7 or 8, wherein the microwave generator is adapted to be pulsed and operated such that a duration of the illumination is adjusted to the temporal functional dynamics of the biological particle.

10. The system according to any one of the previous claims 7 to 9, wherein the illumination adapter and the microwave detector are positioned either collinear or adjacent to each other, or each one at a side of the biological particle, but not directly confronted.

11. The system according to any one of the previous claims 7 to 10, wherein the filter element is a high-pass frequency filter.

12. The system according to any one of the previous claims 7 to 10, wherein the filter element comprises a matched filter receiver configured to exploit signal processing schemes.

**13.** The system according to claim 12, wherein the signal processing schemes are based on Passive Intermodulation Products.

**14.** The system according to any one of the previous claims 7 to 13, wherein the microwave detector is based on sensitive quantum electromagnetic detection schemes.

**Fig. 1**

**Fig. 2**

**FIG. 3**

**FIG. 4**

FIG. 5

FIG. 6A

FIG. 6B

Europäisches
Patentamt

European
Patent Office

Office européen
des brevets

## EUROPEAN SEARCH REPORT

Application Number

EP 21 38 2142

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALZANO QUIRINO: "Proposed test for detection of nonlinear responses in biological preparations exposed to RF energy", BIOELECTROMAGNETICS, vol. 23, no. 4, May 2002 (2002-05), pages 278-287, XP055825126, US ISSN: 0197-8462, DOI: 10.1002/bem.10017 * page 278, left-hand column, paragraph 1 - page 279, left-hand column, paragraph 3 * * page 280, left-hand column, paragraph 3 - paragraph 4 * * figure 1 * | 1-14 | INV. G01N22/00 G01N27/22 |
| X | BALZANO QUIRINO: "RF nonlinear interactions in living cells-II: Detection methods for spectral signatures", BIOELECTROMAGNETICS, vol. 24, no. 7, October 2003 (2003-10), pages 483-488, XP055825133, US ISSN: 0197-8462, DOI: 10.1002/bem.10125 * page 483, left-hand column, paragraph 1 - right-hand column, paragraph 2 * * figure 1 * | 1-14 | TECHNICAL FIELDS SEARCHED (IPC)<br><br>G01N |

-----

-----

-/--

The present search report has been drawn up for all claims

| Place of search | Date of completion of the search | Examiner |
|---|---|---|
| The Hague | 23 July 2021 | Baranski, Jörg |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 38 2142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALZANO QUIRINO ET AL: "A doubly resonant cavity for detection of RF demodulation by living cells", BIOELECTROMAGNETICS, vol. 29, no. 2, February 2008 (2008-02), pages 81-91, XP055825132, US ISSN: 0197-8462, DOI: 10.1002/bem.20365 * page 81, left-hand column, paragraph 1 * * page 85, left-hand column, paragraph 2 - page 86, left-hand column, paragraph 1 * | 1-14 | |
| X | BALZANO QUIRINO ET AL: "RF nonlinear interactions in living cells-I: Nonequilibrium thermodynamic theory", BIOELECTROMAGNETICS, vol. 24, no. 7, October 2003 (2003-10), pages 473-482, XP055825129, US ISSN: 0197-8462, DOI: 10.1002/bem.10126 * abstract * | 1-14 | |

TECHNICAL FIELDS
SEARCHED     (IPC)

-/--

The present search report has been drawn up for all claims

| Place of search | Date of completion of the search | Examiner |
|---|---|---|
| The Hague | 23 July 2021 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 38 2142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEROME J W ET AL: "Computational Modeling Evidence of a Nonthermal Electromagnetic Interaction Mechanism With Living Cells: Microwave Nonlinearity in the Cellular Sodium Ion Channel", IEEE TRANSACTIONS ON MICROWAVE THEORY AND TECHNIQUES, PLENUM, USA, vol. 52, no. 8, August 2004 (2004-08), pages 2040-2045, XP011115663, ISSN: 0018-9480, DOI: 10.1109/TMTT.2004.831924 * page 2040, left-hand column, paragraph 2 - page 241, left-hand column, paragraph 3 * * page 2043, right-hand column, paragraph 1 - page 2044, left-hand column, paragraph 4 * | 1-14 | |
| A | US 2020/209169 A1 (SODICKSON DANIEL K [US] ET AL) 2 July 2020 (2020-07-02) * paragraph [0051] - paragraph [0052] * | 1-14 | |
| A | US 5 144 224 A (LARSEN LAWRENCE E [US]) 1 September 1992 (1992-09-01) * the whole document * | 1-14 | TECHNICAL FIELDS SEARCHED (IPC) |
| A | US 2016/320316 A1 (POTHIER ARNAUD [FR] ET AL) 3 November 2016 (2016-11-03) * the whole document * | 1-14 | |
| A | BO YANG ET AL: "Non-Invasive Imaging Method of Microwave Near Field Based on Solid State Quantum Sensing", ARXIV.ORG, CORNELL UNIVERSITY LIBRARY, 201 OLIN LIBRARY CORNELL UNIVERSITY ITHACA, NY 14853, 5 January 2018 (2018-01-05), XP081204011, DOI: 10.1109/TMTT.2018.2812204 * the whole document * | 1-14 | |

The present search report has been drawn up for all claims

| Place of search | Date of completion of the search | Examiner |
|---|---|---|
| The Hague | 23 July 2021 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 21 38 2142

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2021

| Patent document cited in search report | | Publication date | Patent family member(s) | | Publication date |
|---|---|---|---|---|---|
| US 2020209169 | A1 | 02-07-2020 | NONE | | |
| US 5144224 | A | 01-09-1992 | NONE | | |
| US 2016320316 | A1 | 03-11-2016 | EP | 3087380 A1 | 02-11-2016 |
| | | | FR | 3015521 A1 | 26-06-2015 |
| | | | US | 2016320316 A1 | 03-11-2016 |
| | | | WO | 2015097419 A1 | 02-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10352945 B2
- US 10161937 B2

- US 20190125871 A1


### Non-patent literature cited in the description

- **J. P. STRATFORD et al.** Electrically induced bacterial membrane-potential dynamics correspond to cellular proliferation capacity. *PNAS,* May 2019, vol. 116, 19
- The origins of the brain's endogenous electromagnetic field and its relationship to provision of consciousness. *Journal of Integrative Neuroscience,* 11 July 2020
- The Big and the Small: Challenges of Imaging the Brain's Circuits. *Science,* 11 July 2020, https://science.sciencemag.org/content/334/6056/618/tab-article-info
- **A. RAM ; A. W. LO.** Is Smaller Better? A Proposal to Use Bacteria For Neuroscientific Modeling. *Front. Comput. Neurosci.,* 2018, vol. 12
- **V. PIKOV ; X. ARAKAKI ; M. HARRINGTON ; S. E. FRASER ; P. H. SIEGEL.** Modulation of neuronal activity and plasma membrane properties with low-power millimeter waves in organotypic cortical slices. *J. Neural Eng.,* July 2010, vol. 7 (4
- **M. N. SHNEIDER ; M. PEKKER.** Initiation and blocking of the action potential in an axon in weak ultrasonic or microwave fields. *Phys. Rev. E,* May 2014, vol. 89
- **I. JOHNSON et al.** *The Molecular Probes Handbook,* 2010, www.thermofisher.com/es/es/home/references/molecular-probes-the-handbook/mp-handbook-download.html
- **L. N. MILLER ; W. T. BREWER ; J. D. WILLIAMS ; E. M. FOZO ; T. R. CALHOUN et al.** Second Harmonic Generation Spectroscopy of Membrane Probe Dynamics in Gram-Positive Bacteria. *Biophysical Journal,* October 2019, vol. 117 (8), 1419-1428

- **W. C. CHEW.** Waves and fields in inhomogeneous media. *CERN Document Server,* 1995, https://cds.cern.ch/record/1480882
- **C. A. BALANIS.** Advanced Engineering Electromagnetics. John Wiley & Sons, 2012
- **A. ISHIMARU.** Electromagnetic Wave Propagation, Radiation, and Scattering: From Fundamentals to Applications. John Wiley & Sons, 2017
- **J. M. BENARROCH ; M. ASALLY.** The Microbiologist's Guide to Membrane Potential Dynamics. *Trends in Microbiology,* April 2020, vol. 28
- *Does the resting potential of Chara braunii have an electrogenic component?,* 12 December 2020, https://cdnsciencepub.com/doi/abs/10.1139/b73-089
- **G. FRANCESCHETTI ; I. PINTO.** Cell Membrane Nonlinear Response to an Applied Electromagnetic Field. *IEEE Trans. Microwave Theory Techn.,* July 1984, vol. 32 (7), 653-658
- **A. L. HODGKIN ; A. F. HUXLEY.** A quantitative description of membrane current and its application to conduction and excitation in nerve. *J Physiol,* August 1952, vol. 117 (4), 500-544
- **R. FITZHUGH.** Impulses and Physiological States in Theoretical Models of Nerve Membrane. *Biophys J,* July 1961, vol. 1 (6), 445-466
- **E. M. IZHIKEVICH.** Simple model of spiking neurons. *IEEE Transactions on Neural Networks,* November 2003, vol. 14 (6), 1569-1572
- **P. PHILLIPSON.** A Comparative Study of the Hodgkin-huxley and Fitzhugh-nagumo Models of Neuron Pulse Propagation. *I. J. Bifurcation and Chaos,* December 2005, vol. 15, 3851-3866